# EUROPEAN PATENT SPECIFICATION

(11) **EP 1 656 151 B1**
(45) Date of publication and mention of the grant of the patent: **26.09.2007**
(21) Application number: 04769190.2
(22) Date of filing: 02.08.2004
(51) Int. Cl.: A61K 36/074, A61P 25/00

(54) **EFFECTS OF SPORODERM-BROKEN GERMINATION ACTIVATED GANODERMA SPORES ON TREATMENT OF SPINAL CORD INJURY AND PROLIFERATION AND/OR DIFFERENTIATION OF NEURAL STEM CELLS IN INJURED SPINAL CORD**
WIRKUNGEN VON SPORODERM-GEBROCHENEN GERMINATIONSAKTIVIERTEN GANODERMA-SPOREN AUF DIE BEHANDLUNG VON RÜCKENMARKSVERLETZUNGEN UND DIE PROLIFERATION UND/ODER DIFFERENZIERUNG VON NEURALEN STAMMZELLEN IN VERLETZTEM RÜCKENMARK
EFFETS DES SPORES DE GANODERMES A GERMINATION ACTIVEE ET A SPORODERME ROMPU SUR LE TRAITEMENT DE LESIONS DE LA MOELLE EPINIERE ET SUR LA PROLIFERATION ET/OU LA DIFFERENTIATION DES CELLULES SOUCHES NERVEUSES DANS LA MOELLE EPINIERE BLESSEE

(30) Priority: 01.08.2003 US 631809; 08.01.2004 US 752685
(43) Date of publication of application: 17.05.2006
(73) Proprietor: ENHAN TECHNOLOGY HOLDINGS INTERNATIONAL CO., LTD., Mongkok, Kowloon (CN); Chung, Chee-Keung, Mongkok, Kowloon, Hong Kong (CN); TONG, Siu-Kan, Mongkok, Kowloon (CN)
(72) Inventor: CHUNG, Chee-Keung, Mongkok, Kowloon (CN); TONG, Siu-Kan, Mongkok, Kowloon (CN)
(74) Representative: Oser, Andreas
(86) International application number: PCT/IB2004/002772
(87) International publication number: WO 2005/011720

(56) References cited:
- US-B1- 6 316 002
- CHEUNG^A W M W ET AL: "Ganoderma extract activates MAP kinases and induces the neuronal differentiation of rat pheochromocytoma PC12 cells" 15 December 2000 (2000-12-15), FEBS LETTERS, ELSEVIER SCIENCE PUBLISHERS, AMSTERDAM, NL, PAGE(S) 291-296 , XP004337859 ISSN: 0014-5793 cited in the application page 295, paragraph DISCUSSION

## Description

### FIELD OF THE INVENTION

The present invention relates to a use of sporoderm-broken germination activated ganoderma spores (GASP) for the preparation of a medicament for treating a mammal having spinal cord injury. The GASP are particularly effective in promoting regeneration of the axons and improving survival of the motor neurons within the spinal cord. The present invention also relates to the aforementioned use for promoting proliferation and/or differentiation of neural stem cells in a mammal, particularly after the mammal has a spinal cord injury, by administering to the mammal sporoderm-broken germination activated ganoderma spores (GASP).

### BACKGROUND OF THE INVENTION

The spinal cord coordinates the body's movement and sensation to and from the brain. It is a complex organ containing nerve cells (also known as neurons), and supporting cells (also known as neuroglial cells or glial cells). A typical neuron consists of a cell body, containing the nucleus and the surrounding cytoplasm (perikaryon); several short radiating processes (dendrites); and one long process (the axon), which terminates in twiglike branches (telodendrons) and may have branches (collaterals) projecting along its course. The axon together with its covering or sheath forms the nerve fiber. The neuroglial or glial cells form the supporting structure of nervous tissue. There are three types of glial cells, which are astrocytes, oligodendrocytes, and microglia. Astrocytes and oligodendrocytes (collectively macroglia) are of ectodermal origin. These cells far outnumber neurons in the brain and spinal cord and perform many essential functions. The oligodendrocyte creates the myelin sheaths that insulate axons and improve the speed and reliability of nerve signal transmission. Astrocytes, large star-shaped glial cells, regulate the composition of the fluids that surround neurons. Some of these cells also form scar tissue after injury. Microglia are of mesodermal origin. They are smaller cells that become activated in response to injury and help clean up waste products. All of these glial cells produce substances that support neuron survival and influence axon growth.

There are several types of neurons that lie within the spinal cord and carry out the spinal cord functions. Large motor neurons have long axons that control skeletal muscles in the neck, torso, and limbs. Sensory neurons (also called dorsal root ganglion cells) have axons which carry information from the body into the spinal cord. Spinal interneurons, which lie completely within the spinal cord, help integrate sensory information and generate coordinated signals that control muscles.

Many axons in the spinal cord are covered by sheaths of an insulating substance called myelin, which gives them a whitish appearance; therefore, the region in which they lie is called "white matter." The neurons themselves, with their tree-like dendrites that receive signals from other neurons, make up "gray matter." This gray matter lies in a butterfly-shaped region in the center of the spinal cord. Like the brain, the spinal cord is enclosed in three membranes (meninges): the pia mater, the arachnoid, and the dura mater. The spinal cord is then surrounded by rings of bone called vertebra.

The spinal cord and the brain together make up the central nervous system (CNS). Unlike neurons of the peripheral nervous system (PNS), which carry signals to the limbs, torso, and other parts of the body, it is generally believe that neurons of the CNS do not regenerate after injury. (Walker, New Eng. J. Med., (1991) 324: 1885-1887). Recent research, however, supports that the injured spinal cord can be successfully regenerated, provided that the damaged neurons must be able to survive the injury or be replaced, and the axons must be able to regrow and find appropriate targets. Axons and their targets must then interact to construct synapses, the specialized structures that act as the functional connections between neurons.

The spinal cord is about 18 inches long and extends from the base of the brain, down the middle of the back, to about the waist. The spinal cord is organized into segments along its length. The spinal nerves that branch out from the spinal cord to the other parts of the body are lower motor neurons (LMNs). Thirty-one pairs of spinal nerves exit and enter at each vertebral level and communicate with specific areas of the body. The segments in the neck, or cervical region, referred to as C1 through C8, control signals to the neck, arms, and hands. Those in the thoracic or upper back region (T1 through T12) relay signals to the torso and some parts of the arms. Those in the upper lumbar or mid-back region just below the ribs (L1 through L5) control signals to the hips and legs. Finally, the sacral segments (S1 through S5) lie just below the lumbar segments in the mid-back and control signals to the groin, toes, and some parts of the legs. The effects of spinal cord injury at different segments reflect this organization.

Neurons of the brain and spinal cord respond to signals differently from most other cells of the body, including those in the peripheral nervous system (PNS). The brain and spinal cord (i.e., the CNS) are confined within bony cavities that protect them, but also render them vulnerable to compression damage caused by swelling or forceful injury. Cells of the CNS have a very high rate of metabolism and rely upon blood glucose for energy. The "safety factor," that is the extent to which normal blood flow exceeds the minimum required for healthy functioning, is much smaller in the CNS than in other tissues. For these reasons, CNS cells are particularly vulnerable to reductions in blood flow (ischemia). Other unique features of the CNS are the "blood-brain-barrier" and the "blood-spinal-cord barrier." These barriers, formed by cells lining blood vessels in the CNS, protect neurons by restricting entry of potentially harmful substances and cells of the immune system. Trauma may compromise these barriers, perhaps contributing to further damage in the brain and spinal cord. The blood-spinal-cord barrier also prevents entry of some potentially therapeutic drugs. Finally, in the brain and spinal cord, the glial cells and the extracellular matrix (the material that surrounds cells) differ from those in peripheral nerves. Each of these differences between the PNS and CNS contributes to their different responses to injury.

Although spinal cord is well protected, spinal cord injury does occur due to causes such as trauma (e.g., car accident, violence, falls, sports) and disease (e.g., polio, spina bifida, Friedreich's Ataxia). In the United States, approximately 450,000 people live with spinal cord injury, and there are about 10,000 new cases every year, mostly involving males between ages of 16 to 30. Spinal cord injury can be divided into complete injury (no motor and sensory functions below the level of the injury) and incomplete injury (some functioning below the primary level of injury). Recovery from spinal cord injury is much more difficult because the neurons are very specialized and unable to divide and create new cells.

Usually, injuries to the spinal cord do not result in a cut through the cord; instead, they crush the axons. Researchers studying spinal cords obtained from autopsy have identified several different types of spinal cord injuries. The most common types of spinal cord injuries are contusions (bruising of the spinal cord) and co0mpression injuries (caused by pressure on the spinal cord). Other types of injury included lacerations, caused by a bullet or other object, and central cord syndrome.

The damage that occurs to spinal cord axons within the first few hours after injury is complex and it occurs in stages. The normal blood flow is disrupted, which causes oxygen deprivation to some of the tissues of the spinal cord. Bleeding into the injured area leads to swelling, which can further compress and damage spinal cord axons. The chemical environment becomes destructive, due primarily to the release of highly reactive molecules, e.g., free radicals. These molecules break up cell membranes, including killing cells that were not injured initially. Macrophages that invade the site of injury to clean up debris may also damage uninjured tissue. Non-neuronal cells, such as astrocytes, may divide too often, forming a scar that impedes the regrowth of the injured nerve cell axons.

The early events that follow a spinal cord injury can lead to other kinds of damage later on. Within weeks or months, cysts may form at the site of injury which fill with cerebrospinal fluid. Typically, scar tissue develops around the cysts, creating permanent cavities that can elongate and further damage neurons. Also, nerve cell axons that were not damaged initially often lose their myelin. Over time, these and other events can contribute to more tissue degeneration and a greater loss of function.

Effective drug therapy for spinal cord injury first became a realty in 1990s, when methylprednisolone, the first drug shown to improve recovery from spinal cord injury, moved from clinical trials to standard use. The NASCIS II (National Acute Spinal cord Injury Study II) trial, a multicenter clinical trial comparing methylprednisolone to placebo and to the drug naloxone, showed that methylprednisolone given within 8 hours after injury significantly improves recovery in humans. Completely paralyzed patients given methylprednisolone recovered an average of about 20% of their lost motor function, compared to 8% recovery of function in untreated patients. Partially paralyzed patients recovered an average of 75% of their function, compared to 59% in people who did not receive the drug. Patients treated with naloxone, or treated with methylprednisolone more than 8 hours after injury, did not improve significantly more than patients given a placebo. (National Institutes of Health, "Spinal Cord Injury: Emerging Concepts," (1996) www.ninds.nih.gov/health_and_medical/pubs). The results of the clinical trial of methylprednisolone revolutionized thinking that there is a window of opportunity for acute treatment of spinal cord injury.

Today, more drugs are being tested for treatment of spinal cord injury. For example, U.S. Patent No. 6,291,493 discloses the use of clomethiazole (5-(2-chloroethyl)-4-methylthiazole) for treating pathological conditions caused by compression to the spinal cord, whether traumatically induced or due to compression caused by tumours, haemorrhage, infectious processes or spinal stenosis. U.S. Patent No. 6,288,069 discloses the use of 9-substituted hypoxanthine derivatives (such as N-4-carboxyphenyl-3-(6-oxohydropurin-9-yl) propanamide) to stimulate regeneration or survival of a mammalian motor neuron or of a mammalian sensory neuron. U.S. Patent No. 6,143,714 discloses the use of a hepatocyte growth factor (HGF) to promote the survival, growth and differentiation of motor neurons.

More recently, findings that neurons can be renewed in certain regions of the adult CNS, e.g., in the olfactory bulb, where signals from neurons from the organ of smell reach the brain and in the dentate gyrus of hippocampus, have been reported. Since neurons are unable to divide, the addition of new neurons suggested the existence of immature cells, i.e., progenitor or stem cells, which may generate neurons.

Stem cells are undifferentiated cells capable of proliferation, self-maintenance, and production of a large number of differentiated, functional progeny, regenerating tissue after injury. Stem cells isolated from the early embryonic blastula, i.e., prior to gastrulation, can produce cell types of all different lineages (Keller, Curr. Opin. Cell Biol., 7:862-869 (1995)). Stem cells in adults, however, play the role of replacing cells which have been lost by natural cell death, injury or disease. When transplanted into the brain, these neural stem cells survived and differentiated into neurons and neuroglial cells. (Johansson et al., Cell (1999), 96(1): 25-34). There are also certain numbers of neural stem cells in the spinal cord. They are proliferating cells in the ependyma of the spinal central canal in adult mammals. (Frisen et al., J. Cell Biol. (1995), 131:453-464; Yamamoto et al., Exp. Neurol. (2001), 172:115-127)). A method for isolation of ependymal neural stem cells have recently been described in U.S. patent 6,541,247.

The existence of neural stem cells in the adult mammalian CNS was first demonstrated by culturing cells from the adult rat brain and spinal cord. Under certain culture conditions, a population of multipotent neural stem cells can be propagated. (Reynolds et al., Science (1992) 255:1707-1710). Under these conditions, single cells proliferate *in vitro* and the progeny forms a cluster of aggregated cells. Such cell clones detach from the culture dish after a few days *in vitro*. The cells continue to proliferate and form a characteristic spheroid cell aggregate, referred to as a neurosphere, of tightly clustered cells, all of which are derived from a single cell. Most of the cells in the neurosphere express nestin, an intermediate filament found in neuroepithelial stem cells, (Lendahl et al., Cell (1990), 60:585- 595), but not markers typical for differentiated cells. These undifferentiated cells rapidly differentiate if plated on an adhesive substrate or if serum is added to the culture medium. Importantly, a clone of cells derived from a single cell can generate neurons, astrocytes and oligodendrocytes, demonstrating that at least the initial cell was multipotent (Reynolds et al., Science, *ibid*.). Moreover, if a cell clone is dissociated, many of the cells will form new clusters of undifferentiated multipotent cells, thus fulfilling the criteria for being stem cells.

The development of the mammalian CNS begins in the early stage of fetal development and continues until the post-natal period. (U.S. Patent No. 6,497,872). The first step in neural development is cell birth, which is the precise temporal and spatial sequence in which stem cells and stem cell progeny (i.e., daughter stem cells and progenitor cells) proliferate. Proliferating cells will give rise to neuroblasts, glioblasts and new stem cells.

The second step is a period of cell type differentiation and migration when undifferentiated progenitor cells differentiate into neuroblasts and gliolblasts which give rise to neurons and glial cells and migrate to their final positions. Cells which are derived from the neural tube give rise to neurons and glia of the CNS, while cells derived from the neural crest give rise to the cells of the peripheral nervous system (PNS). Certain factors present during development, such as nerve growth factor (NGF), promote the growth of neural cells. NGF is secreted by cells of the neural crest and stimulates the sprouting and growth of the neuronal axons.

The third step in development occurs when cells acquire specific phenotypic qualities, such as the expression of particular neurotransmitters. At this time, neurons also extend processes which synapse on their targets. Neurons are generated primarily during the fetal period, while oligodendrocytes and astrocytes are generated during the early post-natal period. By the late post-natal period, the CNS has its full complement of nerve cells.

The final step of CNS development is selective cell death, wherein the degeneration and death of specific cells, fibers and synaptic connections "fine-tune" the complex circuitry of the nervous system. This "fine-tuning" continues throughout the life of the host. Later in life, selective degeneration due to aging, infection and other unknown etiologies can lead to neurodegenerative diseases.

There is increasing evidence that nervous system injuries may affect stem cells in the adult CNS. After both spinal cord and brain injuries, nestin expression is increased in cells lining the central canal and in the subventricular zone, respectively. (Frisen et al., J. Cell Biol., *Ibid*.). These cells may be derived from stem cells. With time, nestin expressing cells are seen progressively further from the central canal and the lateral ventricle and these cells express astrocytic markers. (Frisen et al., J. Cell Biol., ibid.). These data demonstrate that stem cells or progenitor cells residing by the ventricular system are induced to proliferate, migrate toward the site of the injury and differentiate to astrocytes.

Currently, no methods are available in clinical practice to stimulate generation of new cells in the nervous system. Transplantation of cells from human embryos or animals have been tested clinically with some encouraging results. However, these methods have several problems, mainly ethical and immunological, which makes it very unlikely that they will be used in any larger number of patients. Accordingly, the discovery of a proliferation and differentiation factor on neural stem cells in the adult CNS of mammals is important and may make it possible to develop strategies to stimulate generation of new neurons or glial cells.

Recently, Cheung et al., FEBS Lett. (2000), 486: 291-296, disclose that *Ganoderma lucidum* extract induces neuronal differentiation *in vitro,* using a primary neuronal cell system (i.e., PC12 cells). The PC12 cells are derived from rat pheochromocytoma cells which respond to the nerve growth factor (NGF). The *Ganoderma lucidum* extract described by Cheung et al. is made from the fruit bodies of the *Ganoderma lucidum.* In the invention to be presented in the following sections, a novel use of the sporoderm-broken germination-activated Ganoderma spores (GASP) from *Ganoderma lucidium* for treatment of spinal cord injury is undertaken. The GASP have previously been disclosed for use in treating patients with cancer, AIDS, hepatitis, diabetes, and cardiovascular diseases, and can prevent or inhibit free radical oxidation and hepatotoxic effects. See U.S. Patent Nos. 6,316,002 and 6,468,542. Unlike methylprednisolone, which has to be given to patients within 8 hours after injury to be effective, the GASP can be administered to humans and animals in a much later time while still achieving significant improvement. A further benefit of using the GASP is that they are non-toxic so that higher dosage can be prescribed to the patients.

In addition, a novel use of the GASP as an effective, safe and practical alternative to induce the proliferation of self neural stem cells of the injured CNS and migration to the injured area and further differentiation into neurons for replacement of damaged or lost neurons is described.

### SUMMARY OF THE INVENTION

The present invention provides a use according to claim 1. The medicament is for treating spinal cord injury by administering an effective amount of germination activated Ganoderma spores (GASP) into a mammal, preferably human, after a spinal cord injury. Gandoderm (*Ganoderma lucidum Leyss* ex Fr. Karst) is a polyporous fungus. It belongs to the class of Basidiomycetes, the family of Polypolaceae, and the genus of *Ganoderma*. The GASP are sporoderm-broken. The GASP are preferred to be orally administered to the mammal within about 1 day of said spinal cord injury.

The causes of the spinal cord injury include compression or severance of the spinal cord (such as severance of the ventral root of the spinal cord, or severance or crush of the sciatic nerve), trauma (such as car accident, violence, falls, sports etc.), or a disease (such as polio, spina bifida, or Friedreich's Ataxia). In addition, the spinal cord injury can be due to damage or death of neurons within the injured spinal cord or crush of axons within the injured spinal cord.

The GASP are preferred to be orally administered to a mammal, including a human, in the amount of about 0.5-15 g per kg of body weight per day, most favorably about 8 g per kg of body weight per day.

By the use of the present invention, survival of neurons, particularly motor neurons, after a spinal cord injury is improved by administering an effective amount of the GASP to a mammal, preferably a human, whose spinal cord has been injured. The GASP are sporoderm-broken. It is preferred that the GASP is administered to the mammal within 1 day of the spinal cord injury. The effective amount of the GASP is about 0.5-15 g per kg of body weight per day, preferably 8 g per kg of body weight per day.

In another embodiment, the above-mentioned use is provided for promoting axon regeneration after a spinal cord injury, which requires the administration, preferably oral administration of an effective amount of the GASP to a mammal, preferably a human, who has a spinal cord injury. The GASP are sporoderm-broken. The GASP are preferred to be orally administered to the mammal within 1 day after the spinal cord injury. The GASP are preferred to be administered to the mammal in the amount of about 0.5-15 g per kg of body weight per day, most favorably about 8 g per kg of body weight per day.

In yet another embodiment, the above-mentioned use is provided for promoting proliferation and/or differentiation of neural stem cells in a mammal. The method requires the administration of an effective amount of sporoderm-broken, germination activated Ganoderma spores (GASP) into a mammal, preferably human. The GASP is especially effective on promoting proliferation and/or differentiation of neural stem cell after the mammal has a spinal cord injury. The causes for a spinal cord injury include, but are not limited to, compression or severance of the spinal cord, trauma (such as car accident, violence, falls, sports etc.), or a disease (such as polio, spina bifida, or Friedreich's Ataxia). In addition, the spinal cord injury can be due to damage or death of neurons within the injured spinal cord or crush of axons within the injured spinal cord. The GASP are preferred to be orally administered to a mammal, including a human, in the amount of about 0.5-15 g per kg of body weight per day, most favorably about 8 g per kg of body weight per day.

### BRIEF DESCRIPTION OF DRAWINGS

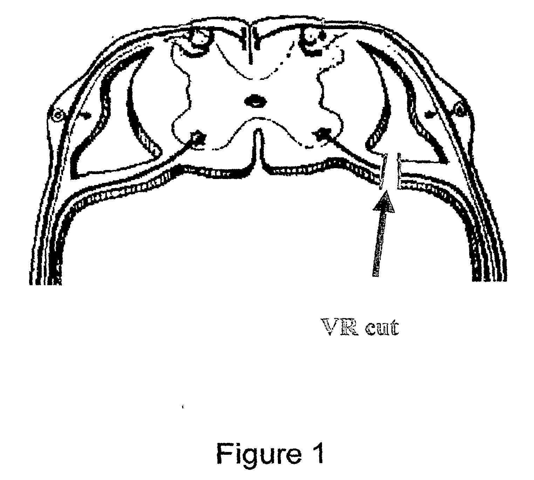

Figure 1 shows the ventral root cut-off site in the right side L3, L4, and L5 nerves of the animal model used in Examples 1 and 2.

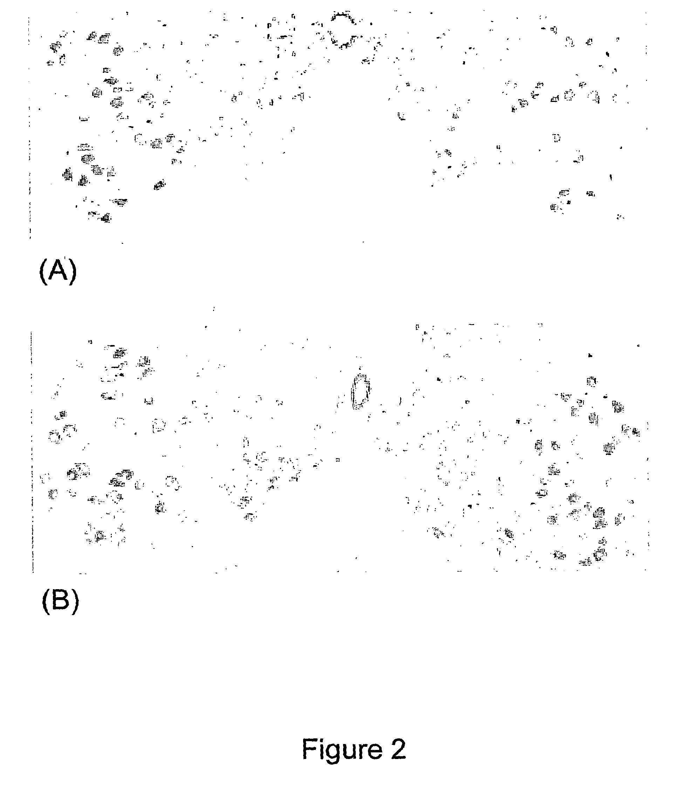

Figure 2 shows the images of the spinal cord stained with neutral red 35 days after the right side ventral roots were cut-off. Survival motor neurons were stained red and showed up as purplish spots against the blue background in these images. As shown in the control group (A), the number of survival motor neurons in the injured (right) side was markedly less than that of the uninjured (left) side. As shown in the GASP treatment group (B), the number of the survival motor neurons at the injured (right) and uninjured (left) sides was statistically insignificant, indicating that the GASP were effective in promoting lumbar motor neuron survival after a spinal cord injury due to cut-off of the ventral roots.

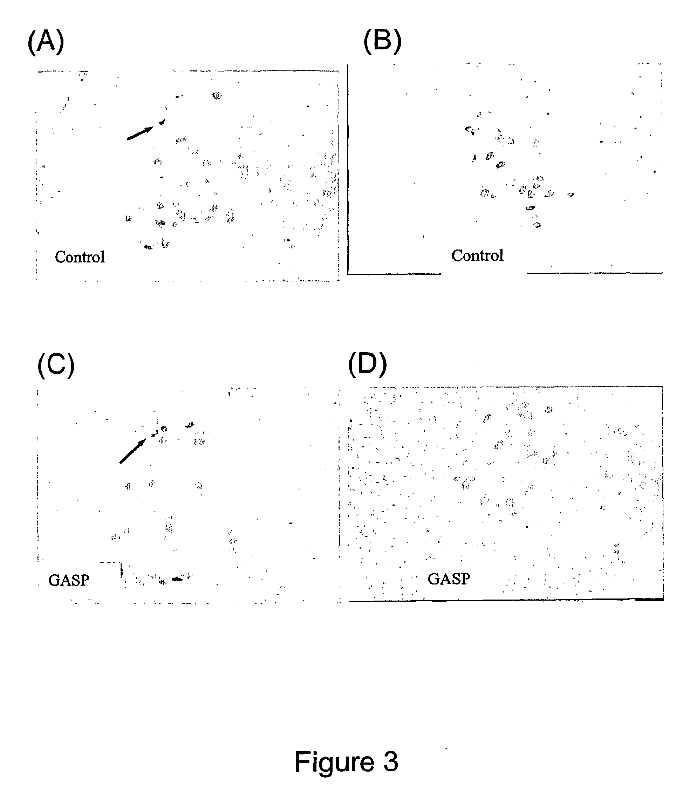

Figure 3 shows the images of the nerve tissues, which were first stained with nicotinamide adenine dinucleotide phosphate-diaphorase (NADPH-d) and re-stained with neutral red. Images (A) and (B) are nerve tissues of rats in the control group at 14 and 35 days after ventral root cut-off, respectively. Images (C) and (D) are nerve tissues of rats in the GASP treatment group at 14 and 35 days after the cut-ff of the ventral roots, respectively. Survival motor neurons were stained with neutral red. Arrows in images (A) and (C) are directed to neurons that were positive for neuronal nitric oxide synthase (nNOS) activity and were stained in a purple color. At 14 days after the cut-off of the ventral roots, there were a high number of nNOS-positive neurons in the nerve tissues of the GASP treatment group (C) than the control group (A), indicating that the GASP were effective in promoting motor neuron survival after spinal cord injury.

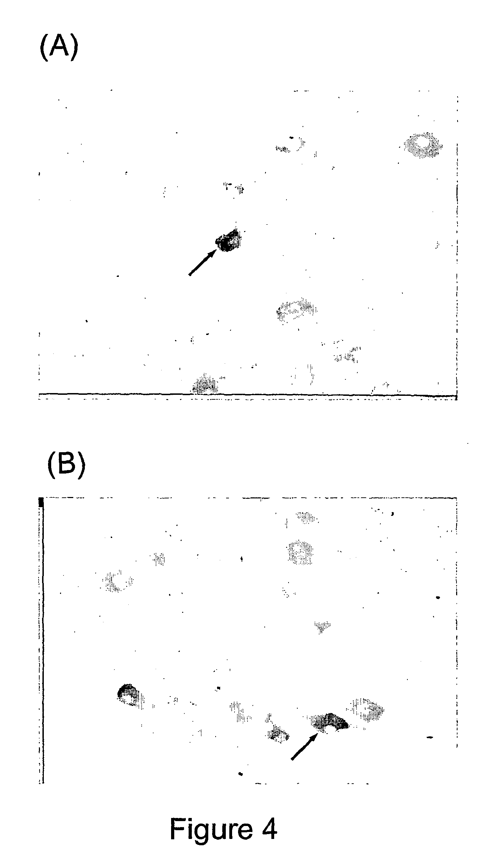

Figure 4 shows the images of nerve tissues (at 100X magnification), which were first stained with NADPH-d, and then re-stained with neutral red. Images (A) and (B) are nerve tissues of rats in the control and GASP treatment groups at 14 days after the cutoff of the ventral roots, respectively. Survival motor neurons were stained with neutral red. Arrows in the images are directed to neurons that were positive for nNOS activity and were stained in a purple color.

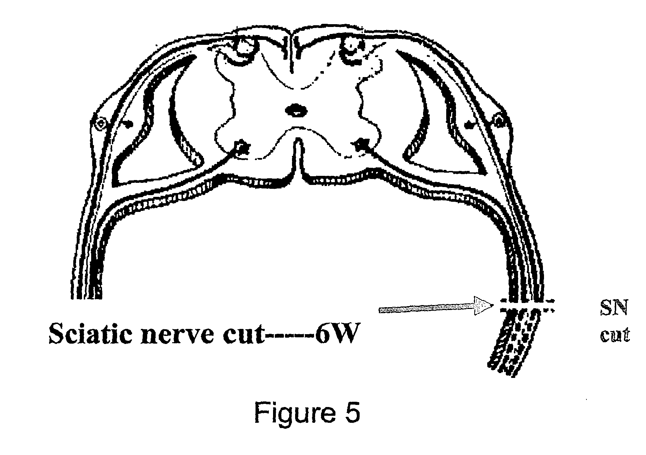

Figure 5 shows the cut-off site in the right side sciatic nerve of the animal model used in Examples 3-6.

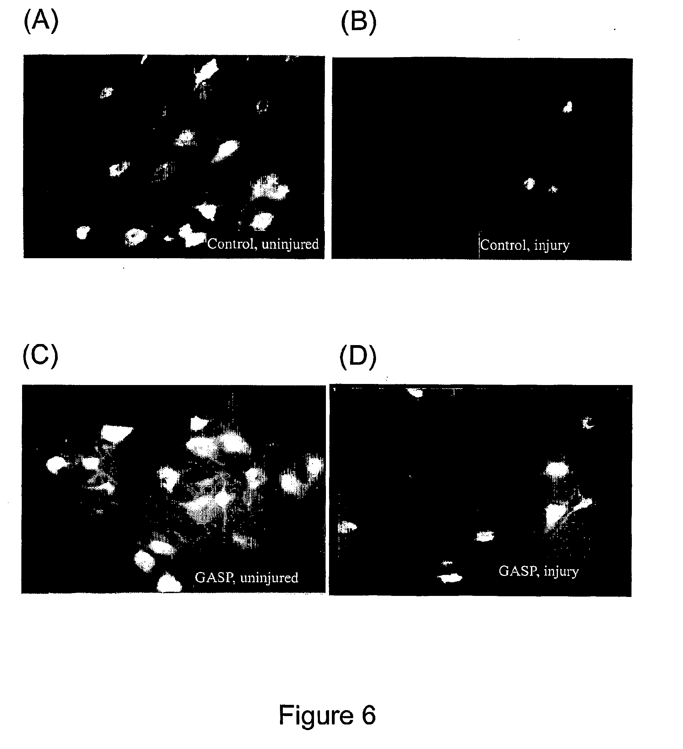

Figure 6 are the images of L4 spinal cord sections labeled with a retrograde tracer Fluorogold (FG) after the right side sciatic nerve was cut-off, followed by perineurium layers re-stitched. Images (A) and (B), respectively, show the uninjured (left) and injured (right) sides of the control group. Images (C) and (D), respectively, show the uninjured (left) and injured (right) sides of the GASP treatment group. The numbers of FG-labeled neurons at the uninjured sides of both groups (see (A) and (C)) were compared. The FG-labeled neurons in the injured side of the control group (B) appeared to be much less than that of the GASP treatment group (D), indicating that the GASP were effective in promoting the regeneration of the injured motor neuron axons.

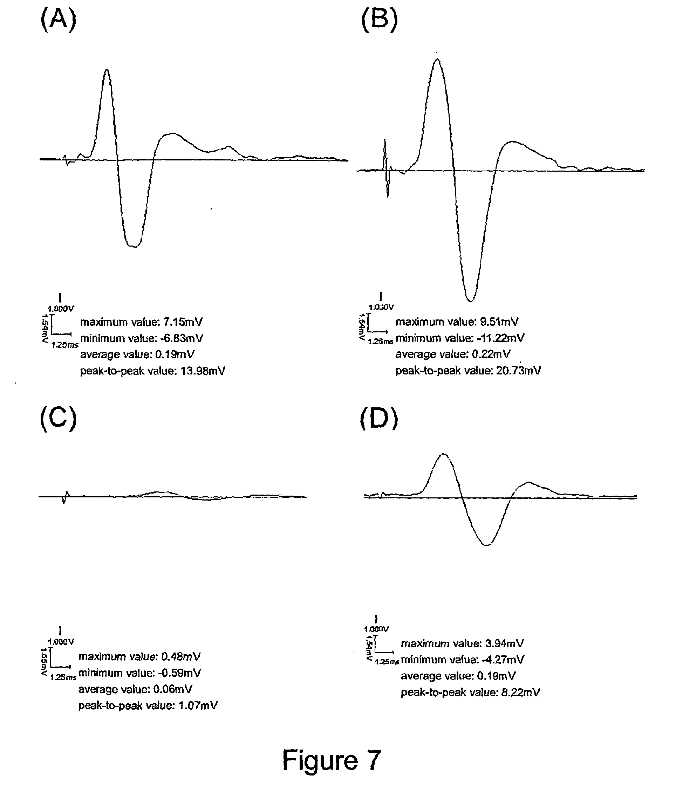

Figure 7 shows the transduction diagrams of the sciatic nerves at the uninjured side (A) and the injured side (C) of the control group and the uninjured side (B) and the injured side (D) of the GASP treatment group, after the right side sciatic nerve was cut-off and re-stitched. The peak-to-peak value and the electrical impulse transduction speed of the electrical impulses were determined. When diagrams (C) and (D) were compared, the peak-to-peak value and the impulse transduction in diagram (D) were higher and faster, indicated that the GASP treatment improved the electrical impulse transduction in the injured sciatic nerve. This suggests that a 6-week GASP treatment was effective in promoting the regeneration of the axons in the injured sciatic nerve.

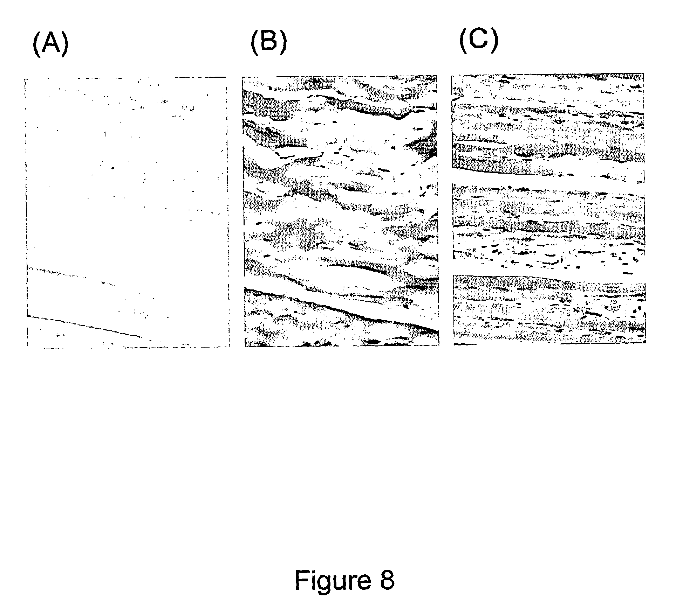

Figure 8 shows the Hematoxylin and Eosin (H&E) stains of normal calf muscle (A); calf muscle of the control group after the sciatic nerve was cut-off for 6 weeks (B); and calf muscle of the GASP treatment group after the sciatic nerve was cut-off for 6 weeks (C). Normal calf muscle tissue exhibited a regular, smooth, and parallel pattern as shown in (A). The calf muscle of the control group (B) exhibited marked irregularity, while the calf muscle of the GASP treatment group (C) retained most of the normal pattern. This suggests that the GASP preserved the calf muscle after the spinal cord injury.

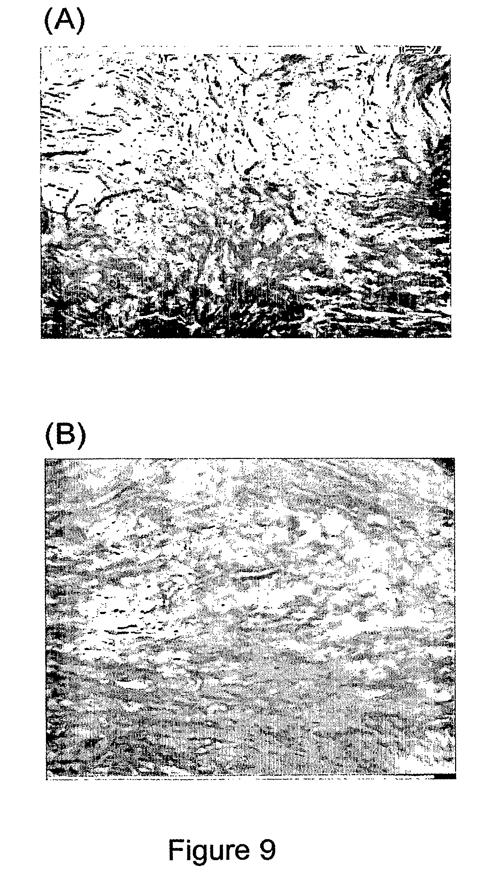

Figure 9 shows the Mallory's stains of repaired sciatic nerve tissues in the control group (A) and the GASP treatment group (B) after the cut-off of the sciatic nerve. Red stain indicated nerve fiber regeneration, which was mostly seen in the nerve tissues of the GASP treatment group (B) and markedly less in that of the control group (A). This indicates that GASP were effective in promoting nerve fiber regeneration.

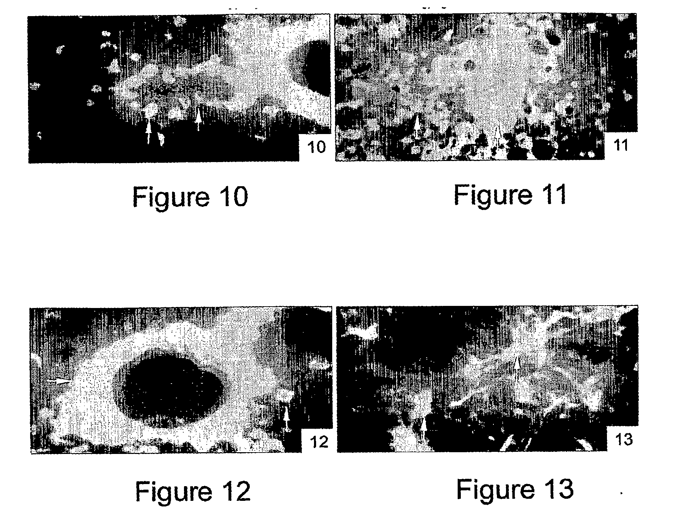

Figure 10 shows the BrdU fluorescent immunohistochemistry of a cross-section of a rat normal spinal cord. Arrow (↑) indicates the BrdU-positive cells in the ependyma of the spinal central canal under fluorescent microscope at 200 fold magnification. Ependyma is the lining membrane of the ventricles of the brain and of the central canal of the spinal cord.

Figure 11 shows the BrdU fluorescent immunohistochemistry of a cross-section of a rat spinal cord 1 day after a spinal cord injury. Arrow (↑) indicates the BrdU-positive cells in the ependyma of the spinal central canal and the surrounding areas under fluorescent microscope at 200 fold magnification.

Figure 12 shows the nestin fluorescent immunohistochemistry of a cross-section of a rat spinal cord 1 day after a spinal cord injury. Arrow (↑) indicates nestin-positive cells in the ependyma of the spinal central canal and the surrounding areas under fluorescent microscope at 200 fold magnification.

Figure 13 shows the nestin fluorescent immunohistochemistry of a cross-section of a rat spinal cord 4 weeks after a spinal cord injury. Arrow (↑) indicates the nestin-positive cells in the injured white matter of the injured/treatment group under fluorescent microscope at 200 fold magnification.

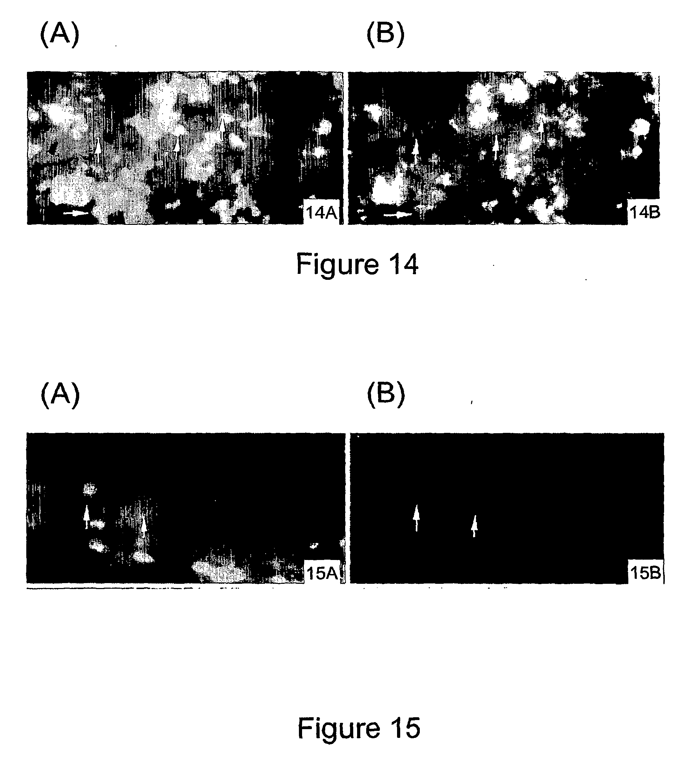

Figure 14A shows the dual BrdU and nestin fluorescent immunohistochemistry of a cross-section of injured spinal cord at 4 weeks after spinal injury. Arrow (↑) indicates the BrdU-positive cells in the white matter of the injured spinal cord of the injured/treatment group under fluorescent microscope at 200 fold magnification.

Figure 14B shows the same view as in Figure 14A. Arrow (↑) indicates that some of the BrdU-positive cells also expressed nestin under fluorescent microscope at 200 fold magnification.

Figure 15A shows a dual BrdU and NF fluorescent immunohistochemistry of a cross-section of injured spinal cord at 4 weeks after spinal injury. Arrow (↑) indicates the BrdU-positive cells in the spinal white matter of the injured/treatment group under fluorescent microscope at 200 fold magnification.

Figure 15B shows the same view as in Figure 15A. Arrow (↑) indicates some of the BrdU-positive cells also expressed NF under fluorescent microscope at 200 fold magnification.

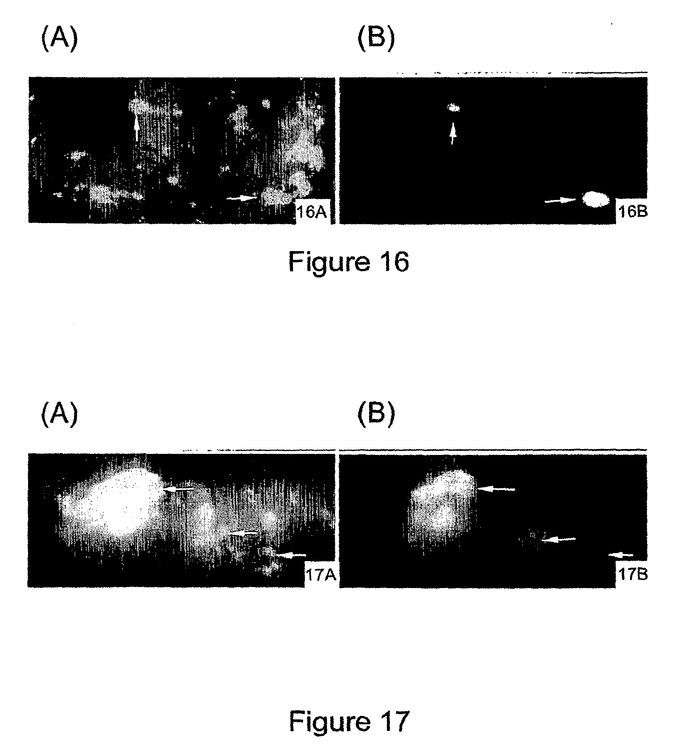

Figure 16A shows the dual BrdU and oligodendrocytin fluorescent immunohistochemistry of a cross-section of injured spinal cord at 4 weeks after spinal injury. Arrow (↑) indicates the BrdU-positive cells in the spinal white matter of the injured/treatment group under fluorescent microscope at 200 fold magnification.

Figure 16B shows the same view as in Figure 16A. Arrow (↑) indicates some of the BrdU-positive cells also expressed oligodendrocytin under fluorescent microscope at 200 fold magnification.

Figure 17A shows the dual BrdU and GFAP fluorescent immunohistochemistry of a cross-section of injured spinal cord at 4 weeks after spinal injury. Arrow (↑) indicates the BrdU-positive cells in the spinal white matter of the injured/treatment group under fluorescent microscope at 200 fold magnification.

Figure 17B shows the same view as in Figure 17A. Arrow (↑) indicates some of the BrdU-positive cells also expressed GFAP under fluorescent microscope at 200 fold magnification.

### DETAILED DESCRIPTION OF THE INVENTION

The present invention provides a method for effective treatment of spinal cord injury and a method for promoting the proliferation and/or differentiation of neural stem cells in a mammal, particularly after a spinal cord injury. The method involves administering an effective amount of germination activated Ganoderma spore powder (GASP) into a mammal having spinal cord injury.

Ganoderma (*Ganoderma lucidum Leyss ex Fr. Karst*) is a polyporous fungus which belongs to the class Basidiomycetes, the family Polypolaceae, and the genus Ganoderma. Ganoderma spores are tiny and mist-like spores of 5∼8 µm in sizes which have extremely hard and resilient, double-layer epispores, thus making them difficult to break open. The spores contain high concentrations of many bioactive substances, including, but are not limited to, polyunsaturated fatty acids, polysaccharides, vitamins, sterols, trace minerals, amino acids, and triterpenes. The GASP used in the present invention are sporoderm-brolcen (i.e., the double-layer epispores of the spores are broken so that the bioactive substances within the spores are released), which is produced by the method described in U.S. Patent No. 6,316,002 ("the '002 patent). Through the unique spore-breaking method described in the '002 patent, the bioactive substances within the GASP are recovered in high yields and the functional activities of the bioactive substances are successfully preserved.

As shown below is a general description of the method used in the '002 patent, which leads to the production of the GASP:
I. Soaking to induce germination: Mature and perfect spores of *Ganodenna lucidum* were carefully selected to carry out a soaking process to induce germination. Spores were kept in clear or distilled water, biological saline solution, or other nutritional solutions that could enable the spores of *Ganoderma lucidum* to germinate rapidly. Examples of nutritional solutions include coconut juice or a 1-5% malt extract solution, 0.5-25% extracts of *Ganoderma lucidum* sporocarps or *Ganoderma lucidum* capillitia, 0.1-5% of culture solution containing biotin, 0.1-3% of culture solution containing monobasic potassium phosphate and magnesium sulfate. The choice of solution would depend on the soaking time required, the amount of spores to be processed and other such factors as availability of materials. One or more of the above germination solutions could be used, with the amount added being 0.1-5 times the weight of the spores of *Ganoderma lucidum.* The soaking time can be determined according to the temperature of the water, and usually the soaking was carried out for 30 min to 8 h with the temperature of the water at 20-43°C. Preferably soaking times were 2-4 hours, and temperature of the water was 25-35°C.
II. Activation culture: The spores of *Ganoderma lucidum* were removed from the soaking solution and excess water was eliminated by allowing it to drip. The spores were then placed in a well-ventilated culturing box at a constant temperature and humidity so that spore activation culture could be carried out. The relative humidity of the culture was generally set at 65-98%, the culture temperature at 18-48°C and the activation time lasted from 30 min to 24 h. Preferably humidity is 85-97% and temperature is 25-35°C. Using the method provided by the present invention, the activation of spores of *Ganoderma lucidum* reached a rate of more than 95%. During activation, the cell walls of the spores of red *Ganoderma lucidum* were clearly softened such that it was easier to penetrate the cell walls of the spores.
III. Treatment of the epispores: After the germination activation process, the spores were treated by enzymolysis. This process was carried out at a low temperature and under conditions such that enzyme activity was maintained, using chitinase, cellulase, or other enzymes, which are commonly used in the industry. The process was complete when the epispores lost their resilience and became brittle. Alternatively, physical treatments were carried out to penetrate the cell walls, for example, micronization, roll pressing, grinding, super high pressure microstream treatment, and other mechanical methods commonly used in the industry could be carried out, with a penetration rate of over 99%.
IV. Drying or extraction: Drying was carried out at low temperature using standard methods including freeze-drying or vacuum-drying etc., which are commonly used in the industry. The obtained product had a moisture content less than 4%. After drying, the bioactive substances were extracted by water or alcohol, or by thin film condensation. The extracted bioactive substances could be further purified by dialysis to ensure no contamination in the final products.
V. Pharmaceutical formulations of the bioactive substances: The bioactive substances can then be made into purified powders, extract pastes, solutions for injection, or for oral consumption. The invention also encompasses the manufacture of pharmaceutical preparations of the active substances, using well-known expedients and methods of manufacture known in the art. In addition, the bioactive substances can be dosed by any convenient method including tablets, capsules, solutions, suppositories, nasal sprays, paranterals, or injection devices. The choice of method for administration is determined by the clinical situation of the patient. The bioactive substances of the present invention, produced by the methods described, include active genes, inducers of the biotic potential promotor, inducers of the multicellular activator, inducers of interferon, lactone A, ganoderma polysaccharide, ganoderma spore fatty acids, ganoderma spore long chain alkyl hydrocarbon, ganoderma triterpenes, sterols, superoxide dismutase, vitamin E, active glycoprotein, certain growth factors, ganoderma acid A, superoxide dismutases (SOD), active glycoproteins, multiple active enzymes, and growth factors and so on. These bioactive substances, in a whole, contribute to the therapeutic uses described in the later sections.

GASP are non-toxic. The preferred method for administering GASP is through oral uptake. Currently, GASP are approved by the Food and Drug Administration (FDA) to be used as dietary supplement in the capsule form under the name of Enhanvol® and Holistol, sold by Enhan Technology Holdings International Co., Ltd. in Hong Kong. Each capsule of GASP contains 0.3 g of GASP. The recommended dosage of GASP, when used as dietary supplement, is 4 times every day, 4 capsules each time. Thus, for an adult of 60 kg, the daily dosage of GASP as dietary supplement is at about 0.08 g/kg of body weight per day.

It has been shown, however, that no physiological and pathological abnormalities were found when 8 g/kg/day of GASP were given to patients and animals. 0.5 g to 15 g/kg/day of GASP have been given to animals and demonstrated significant effects on treatment of spinal cord injury. However, it is understood that the dosage for any particular patient depends upon a variety of factors, including age, body weight, general health, sex, diet, time of administration, route of administration, rate of excretion, drug combination and the severity of the disease. For these reasons, dosing is left to the discretion of the skilled *clinician*.

The present invention is conducted using a rat model. According to Metz et al., J Neurotrauma (2000), 17(1):1-17), the functional, electrophysiological and morphological outcome parameters following spinal cord injury in rats can be extrapolated for those in humans. Metz et al. collected data from human patients with chronic spinal cord injury and compared them to those of rats with contusion spinal cord injury induced by a weight-drop. The results suggest an analogous relationship in rats and humans with respect to functional, electrophysiological, and morphological outcomes, which demonstrates that rat can serve as an adequate animal model for research on functional and morphological changes after spinal cord injury and the effects of new treatment strategies.

In the present invention, five (5) tissue stains and five (5) fluorescent neuronal markers were used. The five tissue stains are neutral red (*See e*.*g*., Figures 2, 3, and 4), nicotinamide adenine dinucleotide phosphate-diaphrase (NADPH-d) (*See e*.*g*., Figures 3 and 4), Fluorogold (See e.g., Figure 6), Hematoxylin & Eosin (See e.g., Figure 8), and Mallory's stain (See e.g., Figure 9). The five fluorescent neuronal markers are BrdU, nestin, neurofilament (NF), oligodendrocytin, and glial fibrillary acidic protein (GFAP). The fluorescent neuronal markers are used to explore the proliferation of cells in general (based on the staining of BrdU), the undifferentiated neural stem cell (based on the staining of nestin), the newly generated neurons from neural stem cell (based on the staining of NF), the newly generated oligodendrocytes from neural stem cell (based on the staining of oligodendrocytin), and the newly generated astrocytes (based on the staining of GFAP).

The following experimental designs are illustrative, but not limiting the scope of the present invention. Reasonable variations, such as those occur to reasonable artisan, can be made herein without departing from the scope of the present invention.

### Example 1.

### Effects of GASP on Survival of Lumbar Motor Neurons

### Animal Model:

Healthy SD rats supplied by the Guangdong Medical Animals Center with body weights of approximately 100 g were selected.

### Method:

The SD rats were divided into 2 groups, i.e., the GASP treatment group and the control group. In each rat, the right side of the ventral roots at the L3, L4, and L5 lumbar nerves was cut off. (*See* Figure 1). The left side of the ventral roots was left untouched to be used for comparison.

GASP (20 g) was dissolved in 100 mL of 0.5% sodium carboxymethyl cellulose to a final concentration of 20%. After the ventral roots were cut off for about one day, the rats in the GASP treatment group were fed approximately 2 mL of GASP solution twice daily via gavage for 35 days. Daily dose of GASP was 8 g/kg/day. The rats in the control group were fed the same amount of 0.5% sodium carboxymethyl cellulose twice daily for 35 days. All rats were kept under the same condition.

At the end of the treatment period, nerve tissues were collected from both groups. Segments of spinal cord were fixed using 4% paraformaldehyde, soaked in a 30% sugar cane in phosphate buffered saline (PBS) solution, and frozen-sectioned to slices of 30 µm thickness. Every fifth slice was selected and a total of 10 slices were stained with neutral red. The survival neurons were stained red in the sectioned slices and were counted according to the method of Clarke and Oppenheim. For each rat, the survival motor neurons on the 10 nerve slices were averaged. ,

Statistical analysis was performed using T-test.

### Results:

The results are shown in Table 1 and Figure 2.

**Table 1. Effects of GASP on Motor Neurons Survival After the Cut-off of Lumbar Ventral Roots**

| | Control group (n=6) | GASP treatment group (n=7) | *P** |
|---|---|---|---|
| Number of survived motor neurons at the uninjured (left) side | 15.02 ± 0.86 | 15.45 ± 0.81 | > 0.05 |
| Number of survived motor neurons at the injured (right) side | 6.43 ± 1.49 | 13.61 ± 0.74 | |
| *P*** | <0.01 | <0.01 | |
| %survival*** at the injured (right) side | 56.88 ± 3.20 | 89.88 ± 2.24 | <0.01 |

| | | | |
|---|---|---|---|
| * *p* value denotes comparison between the control and the GASP treatment groups ** *p* value denotes comparison of numbers of neurons at the uninjured (left) side and the injured (right) side within the same group *** % survival was determined as the percent ratio of the number of neurons at the injured (right) side to that at the uninjured (left) side. | | | |

The results show that the numbers of survived motor neurons at the uninjured sides in both groups were similar. However, the number of survived motor neurons at the injured side without treatment after the cut-off of the L3, L4 and L5 nerves was significantly lower (6.43 ± 1.49), as compared to the uninjured side (15.02 ± 0.86) and the injured side of the GASP treatment group (13.61 ± 0.74). The % survival rate of the GASP treatment group was 90%, which was significantly higher than that of the control group (57%). Similar findings were observed in the neural red stains of the spinal cord tissues (Figure 2).

These results indicated that 35 days of the GASP treatment at 8 g/kg/day was effective in promoting the survival of lumbar motor neurons to 90% survival in rats after their ventral roots were cut off.

### Conclusion:

It had been demonstrated that GASP was effective in promoting the survival of motor neurons after a severance type of nerve injury.

### Example 2.

### Effects of GASP on Lumbar Motor Neuron Survival

The activity of neuronal nitric oxide synthase (nNOS) in the injured neurons signifies with the survival of the motor neurons. The histochemical nicotinamide adenine dinucleotide phosphate-diaphorase (NADPH-d) reaction is considered a suitable marker for nNOS activity. Thus, the measure of the activity of nNOS using the NADPH-d staining method can be used to determine the survival of the motor neurons.

### Method:

Animal models were prepared, grouped and treated as described in Example 1, *supra.*

After 14 days of treatment, five (5) rats from each group were sacrificed and their respective nerve tissues were collected. The remaining rats in each group were continued to be fed with their daily dosage of GASP or sodium carboxymethyl cellulose solution until day 35. These rats were then sacrificed and the nerve tissues were collected.

Segments of spinal cord tissues were fixed using 4% paraformaldehyde, soaked in a phosphate buffered saline solution (PBS) containing 30% sugar cane, and frozen-sectioned to 30 µm thickness for use on slides. The selected nerve tissue sections were incubated in an NADPH solution and incubated for 1-3 hours, washed and then re-stained with 1% neutral red.

Statistical analysis of the treatment and control groups was performed using T-test.

### Results:

The results of the nNOS activity in the nerve tissues are shown in Table 2 and Figures 3 and 4.

**Table 2. Effects of the GASP on Motor Neuron Survival After Lumbar Ventral Roots Being Cut-off for 14 Days**

| | Control group (n=5) | GASP treatment group (n=5) | *P** |
|---|---|---|---|
| Number of survived motor neurons at the uninjured (left) side | 13.69 ± 0.70 | 15.26 ± 0.30 | |
| Number of nNOS-positive neurons at the injured (right) side | 1.41 ± 0.26 | 5.55 ± 0.60 | |
| % nNOS-positive at the injured (right) side ** | 10.36 ± 1.85 | 38.29 ± 4.37 | < 0.01 |

| | | | |
|---|---|---|---|
| * *p* value denotes comparison between the control and the GASP treatment groups. ** % nNOS-positive was determined as the percent ratio of the number of nNOS-positive neurons to that of the survived motor neurons at the injured (right) side of the ventral roots. | | | |

As shown in Table 2 and Figures 3 and 4, at 14 days after the cut-off of the ventral roots, the number of survived motor neurons (stained in red) at the uninjured side of the control group was similar to that of the GASP treatment group. However, the number of nNOS-positive neurons (stained in purple) of the control group was markedly less than that of the GASP treatment group. As shown in Figure 3(B) and 3(D), there were more survived motor neurons in the GASP treatment group (D) than those in the control group (B). The results suggest that the GASP had positive effects on promoting the survival of the motor neurons after the nerves at the ventral roots were cut-off.

### Conclusion:

It had been demonstrated that the GASP were effective in promoting the survival of the neurons after a severance type of spinal cord nerve injury.

### Example 3.

### Effects of the GASP on Regeneration of Injured Axons

### Animal Model:

A rat sciatic nerve cut-off/re-stitched model was used in this study. Briefly, a section of the right side sciatic nerve of the experimental animal was exposed and cut off as shown in Figure 5. The epineurium layers of the two open ends of sciatic nerve were then microsurgically stitched together to give an animal model that mimics cut/crush types of injury. The left side sciatic nerve was left untouched to be used for comparison to the injured side.

### Method:

Animal models were prepared as shown in Examples 1-2, *supra,* and divided into 2 groups, the GASP treatment and the control groups.

GASP (20 g) was dissolved in 100 mL of 0.5% sodium carboxymethyl cellulose to a final concentration of 20%. The rats in the GASP treatment group were fed approximately 2 mL of GASP solution twice daily via gavage. Daily dose of GASP was 8 g/kg/day. The rats in the control group were fed the same amount of 0.5% sodium carboxymethyl cellulose twice daily. All rats were kept under the same conditions.

Fluorogold (FG), a retrograde fluorescent tracer, was used to track the regeneration of the axons. 2.5 µL of 3% FG solution were injected using a microinjector into the sciatic nerves at both injured (right) and uninjured (left) sides. At the injured (right) side, FG was injected at 5 mm distal to the site of injury. The L4 spinal cords were then removed from the experimental rats, frozen-sectioned and studied under fluorescence microscope. "FG%" was calculated as the percent ratio of the number of FG-positive neurons at the injured side to that of the uninjured side of the same animal.

Statistical analysis was performed using T test.

### Results:

The results of the axon regeneration studies using FG are shown in Table 3 and Figure 6.

**Table 3. Effects of the GASP on Regeneration of the Injured Axons**

| FG-positive neurons | control group (n=5) | GASP treatment group (n=5) | *P** |
|---|---|---|---|
| Uninjured (left) side | 18.27 ± 0.97 | 16.99 ± 0.96 | > 0.05 |
| Injured (right) side | 3.37 ± 0.38 | 9.75 ± 0.64 | |
| *P*** | < 0.01 | < 0.01 | < 0.01 |
| FG%*** | 17.89 ± 4.41 | 59.41 ± 3.47 | |

| | | | |
|---|---|---|---|
| * p value denotes comparison between the control and the GASP treatment groups ** *p* value denotes comparison of the number of neurons at the uninjured (left) side and the injured (right) side within the same group. *** FG% was determined as the percent ratio of the number of the FG-positive neurons at the injured (right) side to that at the uninjured (left) side. | | | |

FG is a retrograde fluorescence tracer. It was injected at the distal side of the cut-off/re-stitched injury, thus the detection of the FG-labeled neurons in the spinal cord region was indicative to axon regeneration.

As shown in Table 3 and Figure 6, the numbers of FG-labeled neurons at the uninjured sides of both groups were similar, contrasting to the numbers of the FG-labeled neurons at the injured sides of both groups, which were significantly lower. However, the FG-labeled neurons at the injured side of the GASP group demonstrated a significant greater number than those at the injured side of the control group.

Based on counting of the FG-labeled neurons at the injured and the uninjured sides of the sciatic nerves, the regeneration rate (%) of the axons can be determined by dividing the number of the FG-labeled neurons at the injured side of the sciatic nerve by the number of the FG-labeled neurons at the uninjured side of the sciatic nerve in the same animal. Under this calculation, the regeneration rate of the axons was about 18% in the control group and about 59% in the GASP treatment group.

### Conclusion:

It had been demonstrated that the GASP was effective in treating crush injuries of the spinal cord, particularly in promoting the regeneration of the axons.

### Example 4.

### Electrophysiology Effects of the GASP on Regeneration of the Injured Axons

### Method:

The animal models were prepared and treated as described in Example 3, *supra.*

After 6 weeks of treatment with or without GASP, the rats were anaesthetized and the sciatic and sural nerves were exposed. An electrical stimulator was attached mesially to the repaired side and the recording electrode was placed at the distal side. After the electrical stimulation, the electrical impulse traveling along the nerve was traced and recorded. The same procedure was repeated at the uninjured side. Transduction speeds of the injured and uninjured sciatic nerves were determined. The ratio of the transduction speed of the injured side to that of the uninjured side was used as a marker for axon regeneration. The peak-to-peak values of the electrical impulse traveling along the nerves at the injured and uninjured sides were determined and used as an indicator for axon regeneration.

Statistical analysis was performed using T-test.

### Results:

The results are shown in Table 4 and Figure 7.

**Table 4. Electrophysiological Effects of the GASP on Regeneration of Injured Axons**

| peak-to-peak value (mV) | control group (n=5) | GASP treatment group (n=5) | *P** |
|---|---|---|---|
| uninjured (left) side | 14.44 ± 0.14 | 19.30 ± 0.15 | > 0.05 |
| injured (right) side | 0.74 ± 0.01 | 7.75 ± 0.09 | |
| *P*** | < 0.01 | < 0.01 | < 0.01 |
| % | 5.13 ± 0.08 | 40.19 ± 0.51 | |

| | | | |
|---|---|---|---|
| * *p* value denotes comparison between the control and the GASP treatment groups. ** *p* value denotes comparison of the number of neurons at the uninjured (left) side and that of the injured (right) side within the same group. ***"%" was determined as the percent ratio of the peak-to-peak value measured at the injured (right) side to that of the uninjured (left) side. | | | |

After the sciatic nerve was cut off and its outer layers stitched together, the transduction of an electrical impulse alone the nerve across the site of injury was indicative of the regeneration of the nerve axons. As shown in Table 4 and Figure 7(A) and 7(B), the transduction of the electrical impulses at the uninjured sides of both groups were similar in terms of the peak-to-peak values and the transduction speed. However, at the injured side of the control group, the transduction of an electrical stimulation almost completely ceased (Table 4, Figure 7(C)). In comparison, about 40% of the electrical stimulation traveled along the sciatic nerve across the site of injury at a slightly slower speed at the injured side of the GASP treatment group. These results indicated that the GASP treatment at 8 g/kg/day for 6 weeks was effective in promoting axon regeneration in rats after the sciatic nerve was cut-off and the outer layers re-stitched.

### Conclusion:

It has been demonstrated that GASP was effective in promoting axon regeneration in a crush type of spinal cord injury.

### Example 5.

### Effects of GASP on Regeneration of Injured Axons

### Method:

The animal models were prepared and treated as described in Example 3, *supra.*

After 6 weeks of treatment with or without the GASP, the calf muscles (*musculus peronaeus longus*) were removed from both of the injured and uninjured sides of each rat. The isolated muscles were weighed and recorded. The muscle atrophy of the isolated muscles was determined. The isolated muscles were then stained with Hematoxylin and Eosin (H&E stain) for use in histology study.

### Results:

Marked muscle atrophy and weight reduction were observed in the calf muscles isolated from the injured side of the control group. As shown in Figure 8(A), normal calf muscle tissue exhibited a regular, smooth, and parallel pattern. The calf muscle of the control group (Figure 8(B)) exhibited marked irregularity, while the calf muscle of the GASP treatment group (Figure 8(C)) retained most of the normal pattern. This suggests that the GASP had the effects of preserving the calf muscle after the nerve injury.

### Conclusion

It had been demonstrated that the GASP promoted muscle preservation after spinal cord nerve injury.

### Example 6.

### Effects of the GASP on Regeneration of Nerve Fibers in the Injured Axons

### Method:

The animal models were prepared and treated as described in Example 3, *supra.*

After 6 weeks of treatment, the sciatic nerve axons were removed from both the injured and uninjured sides of each rat. The isolated nerve tissues were fixed, sectioned and then stained with Mallory's stain.

### Results:

As shown in Figure 9, red stain indicated nerve fiber regeneration, which was mostly seen in the nerve tissue of the GASP treatment group (Figure 9(B)) and markedly less in that of the control group (Figure 9(A)). This indicates that the GASP was effective in promoting nerve fiber regeneration in rats after the sciatic nerve was cut-off, followed by re-stitching of the outer layers of the sciatic nerve.

### Conclusion

It had been demonstrated that GASP was effective in promoting nerve fiber regeneration after a crush nerve injury.

### Example 7.

### Preparation of Spinal Hemisection Animal Model, Injection of BrdU, and Treatment of GASP

### Animal grouping:

Twenty-six (26) female SD rats (150-180 g) were purchased from the Experimental Animal Center of the Sun Yat-sen University. The animals were separated into 3 groups: the normal control group (2 rats), the injured/control group (12 rats: 2 rats in the 1-day group, 5 rats each in the 2-week and 4-week groups) and the injured/treatment group (12 rats: 2 rats in the 1-day group, 5 rats each in the 2-week and 4-week groups).

### Animal Model preparation:

1% Pentobarbital sodium (35 ∼ 40 mg/kg) was injected intraperitoneally to anesthetize the rats in the injured/control group and the injured/treatment group. The skin on the back of the rat was cut open at the centerline. The thoracic (T) 12 spinal segment was exposed under a surgical microscope and hemisection of the right side of the spinal cord was performed. After surgery, penicillin was injected intramuscularly.

### BrdU injection and treatment:

The animals in all three groups received intraperitoneal injection of BrdU (50 mg/kg) twice a day for consecutive 10 days. After the spinal injury, the animals in the injured/treatment group were administered twice a day via stomach a GASP solution at 8 g/kg/day. The injured/control group was administered via stomach a 5% sodium carboxycellulose solution (i.e., the solvent in the GASP solution). The GASP is provided by Enhan Technology Holdings International Co., Ltd.. The thimidine analogue, 5-BrdU, is commonly used to study DNA synthesis and cell proliferation. It is a nonradioactive molecule. BrdU is substituted stoichiometrically for thymidine in DNA during the S-phase (synthesis) of cell growth where the cellular DNA content is doubling between the G1 and G2 cell phases. A measure of the incorporated BrdU is related to the new DNA content, *i*.*e*., the status or location of the cell in the S-phase. Thus, the detection of BrdU-substituted DNA is functionally related to synthesis of DNA during the S-phase of the cell cycle.

### Example 8.

### Perfusion, Fixation and Sampling

At 1 day, 2 weeks, and 4 weeks after the spinal injury, the animals in the corresponding groups were anesthetized by an intraperitoneal injection of 1% pentobarbital sodium. The chest of the anesthetized animal was opened and a catheter was inserted from the left ventricle to the aorta. The animal was first rapidly perfused with physiologic saline, then followed with perfusion of 4% paraformaldehyde in 0.1 M PBS (pH 7.4) to fix the tissue of the animal. The T8-L1 spinal segment was harvested, placed in a fresh fixation solution and fixed for 4 hours, and then placed in a 30% sucrose solution at 4°C overnight. The T10, T11, T12, T13 and L1 spinal segments were continuously frozen-sectioned at cross sections to slices of 30 µm thickness.

### Example 9.

### Fluorescent Immunohistochemistry

The cross-section slices of spinal cords were first rinsed three times with 0.01 M PBS and each time the rinsing lasted for about 5 minutes. The spinal slices were then soaked in 2 N HCl for 30 minutes at 37°C, followed-by 0.1 M sodium borate buffer (pH 8.3) for 15 minutes and 20.2% Triton X-100 solution at 37°C for 30 minutes. The spinal slices were then incubated in normal sheep serum at 37°C for 20 minutes. Mouse antiBrdU primary antibody was added dropwise and the spinal slices were further incubated at 37°C for 2 hours. The spinal slices were rinsed with 0.01 M PBS three times and each time the rinsing lasted for 5 minutes. The biotinylated secondary antibody was then added dropwise and the spinal slices were incubating at 37°C for 30 minutes. The spinal slices were rinsed with 0.01 M PBS three times and each time the rinsing lasted for 5 minutes. The SABC-cys3 fluorescent complex solution was added dropwise and the spinal slices were incubating at 37°C for 30 minutes. The spinal slices were rinsed with 0.01 M PBS three times while each time the rinsing lasted for 5 minutes. For negative control, the primary antibody was omitted and replaced with PBS. The rest of the procedures were the same as shown above.

After completing the BrdU staining, the spinal slices of each rat were divided into 4, each separately treated with the fluorescent immunohistochemistry stains for nestin (neural stem cell marker), NF (neuron marker), oligodendrocytin (oligodendrocyte marker) and GFAP (astroglial cell marker), respectively.

Under fluorescent microscope at 10 x 20 magnification, the numbers of BrdU-positive cells in the ependyma of the central canal of the cross-sectioned spinal slices were counted. For each rat, ten spinal slices were counted to determine the average number of BrdU-positive cells in the ependyma of the central canal per spinal slice. The calculated results of each group were expressed as the average numbers of BrdU-positive cells in the ependyma of the central canal ± standard deviation. The t-test on the average numbers of two samples was performed.

### Results of Examples 7-9:

### 1. Normal Ependyma in the Spinal Cord Central Canal

In the spinal cross sections of the neck and thoracic regions of the normal control group, the ependymal cells of the central canal appeared as a round shape, but as an oval shape in the lumbar region. For the purpose of locating the proliferation of the ependymal cells, mainly the BrdU-positive cells in the ependyma were counted. This was because most of the BrdU-positive cells were located at the ependyma (Figure 10). Only a few BrdU-positive cells were distributed in the spinal white matter and gray matter. Detection of nestin expression could lead to the determination of the cell counts of the neuron stem cells. The results indicated that only a small number of nestin-positive cells in the ependyma.

### 2. Ependymal Cell Proliferation and Nestin Expression in the Spinal Cord Central Canal after Spinal Hemisection

At 1 day after the spinal injury, the ependymal cell layer of the central canal was thickened. The BrdU-positive cells markedly increased after spinal hemisection (Figure 11) and more numbers of BrdU-positive cells were observed in the injured animals than in the normal control animals. Meanwhile, in the spinal white matter and gray matter, more BrdU-positive cells were observed after spinal hemisection than those seen in the normal control group. This phenomenon was also observed in several spinal segments near the injured site. At 2 weeks after the spinal injury, the numbers of BrdU-positive cells in the ependyma were less than those seen at 1 day after spinal injury but still higher than those of the normal control group at 2 weeks. Meanwhile, the BrdU-positive cells of the animals in the injured/treatment group were more than those of the injured/control group. At 4 weeks after spinal injury, less BrdU-positive cells, but still significantly higher than those of the normal control group, were seen in the ependyma. The numbers of BrdU-positive cells of the injured/treatment group were higher than those of the injured/control group (Table 1). Thus, at 1 day, 2 weeks and 4 weeks after spinal injury, there were BrdU-positive cells observed in the ependyma of the spinal cord central canal. As the time progressed, the number of BrdU-positive cells decreased. The BrdU-positive cells not only existed in the ependyma of the central canal, but also in the spinal white matter and gray matter. The closer to the injured site, the more the BrdU-positive cells in the ependyma were.

**Table 5. Average Numbers of the BrdU-positive cells (x ± s) in the Ependyma of the Spinal Cord Central Canal 4 Weeks After Spinal Hemisection**

| Group | numbers of rats | average numbers of BrdU-positive cells |
|---|---|---|
| Normal control | 2 | 18.17 ± 2.81 |
| Injured/control | 5 | 29.91 ± 3.68 |
| Injured/treatment | 5 | 45.67 ± 3.62 |

| | | |
|---|---|---|
| *t* test: pairwise comparison among the 3 groups, *P* < 0.05 | | |

At 1 day after the spinal hemisection, nestin-positive cells in the ependyma of the central canal increased. There were also nestin-positive cells in the spinal white matter and gray matter (Figure 12). At 2 weeks and 4 weeks after spinal injury, there were only a small number of nestin-positive cells in the ependyma of the central canal while most distributed in the spinal white matter and gray matter. In the spinal segments near the injured site, the white matter ventral to the hemisection injured site had radial nestin-positive protrusions. Also seen in other white matter were nestin-positive cell bodies and their protrusions (Figure 13).

At 2 weeks and 4 weeks after the spinal injury, the ependymal cell layer of the spinal central canal of the injured/treatment group was thickened, having more BrdU-positive cells than those of the injured/control group. From the results of the BrdU and nestin, BrdU and NF, BrdU and oligodendrocytin, and BrdU and GFAP dual immunohistochemical stains, it was found that there were no cells positive with dual stains in the ependyma of the central canal. However, in the spinal white matter of the injured/treatment group there were a small number of cells positive with dual stains of BrdU and nestin (Figures 14A and 14B), BrdU and NF (Figures 15A and 15B), BrdU and oligodendrocytin (Figures 16A and 16B), and BrdU and GFAP (Figures 17A and 17B).

### Discussion:

The above study confirmed that at 1 day after the spinal hemisection, the ependymal cells of the central canal proliferated, but the number of proliferated cells declined as the time progressed. The closer to the spinal injured site, the more the proliferation of the ependymal cells was. Proliferated cells not only existed in the ependymal cells of the central canal, but also in the spinal white matter and gray matter. Meanwhile, some proliferated cells expressed nestin, indicating these cells had differentiated into neural stem cells. These findings agreed with the report of Yamamoto et al., Exp. Neurol., 2001; 172(1):115-127, that neural stem cells not only existed in the ependyma of the spinal central canal, but also existed in other parts of the spinal cord. In addition, the numbers of proliferated ependymal cells of the central canal in rats of the injured/treatment group were higher than those of the injured/control group, indicating GASP have the effects of promoting cell proliferation of the ependymal cells of the central canal in the injured spinal cord.

The results of the present study confirmed that after the spinal injury, a small number of proliferated ependymal cells could differentiate into neural stem cells, neuron-like cells, oligodendrocyte-like cells and astroglial-like cells which possibly involved in the repair of the injured spinal cord.

### Conclusion:

GASP is effective in promoting the proliferation and/or differentiation of neural stem cells in rats having spinal cord injury.

While the invention has been described by way of examples and in term of the preferred embodiments, it is to be understood that the invention is not limited to the disclosed embodiments.

## Claims

1. A use of sporoderm-broken germination activated Ganoderma spores (GASP) for the preparation of a medicament for treating a mammal with spinal cord injury.

2. The use according to claim 1, wherein said GASP is for oral administration to said mammal.

3. The use according to claim 1, wherein said GASP is for administration to said mammal within 1 day of said spinal cord injury.

4. The use according to claim 1, wherein said mammal is human.

5. The use according to claim 1, wherein said spinal cord injury is caused by any one of the following conditions:
compression or severance of the spinal cord; a trauma; severance of a ventral root of the spinal cord; severance or crush of the sciatic nerve; a disease; damage or death of neurons within said injured spinal cord; and crush of axons with said injured spinal cord.

6. The use according to claim 5, wherein said disease is polio, spina bifida, or Friedreich's Ataxia.

7. The use according to claim 5, wherein said neurons are motor neurons.

8. The use according to claim 1, wherein the amount of said GASP for said medicament is 0.5-15g per kg of body weight per day.

9. The use according to any one of the preceding claims for improving survival of neurons after a spinal cord injury.

10. The use according to claim 9, wherein said neuron is a motor neuron in said injured spinal cord.

11. The use according to any one of the preceding claims for promoting axon regeneration in a spinal cord injury.

12. The use according to any one of the preceding claims, wherein the treatment of a mammal with spinal cord injury is for promoting proliferation and/or differentiation of neural stem cells in a mammal.

13. The use according to claim 12, wherein said neural stem cells are differentiated into any one of the following cell types:
neurons; astrocytes; and oligodendrocytes.

## Patentansprüche

1. Verwendung von Sporoderm-gebrochenen, Keimungs-aktivierten Ganoderma-Sporen (GASP) zur Herstellung eines Medikaments zum Behandeln eines Säugers mit Rückenmarksverletzung.

2. Verwendung gemäß Anspruch 1, wobei die GASP zur oralen Verabreichung an den Säuger sind.

3. Verwendung gemäß Anspruch 1, wobei die GASP zur oralen Verabreichung an den Säuger innerhalb 1 Tages der Rückenmarksverletzung sind.

4. Verwendung gemäß Anspruch 1, wobei der Säuger ein Mensch ist.

5. Verwendung gemäß Anspruch 1, wobei die Rückenmarksverletzung durch irgendeine der folgenden Gegebenheiten verursacht wird:
Komprimierung oder Trennung des Rückenmarks; ein Trauma; Trennung einer ventralen Wurzel des Rückenmarks; Trennung oder Quetschung des Ischiasnervs; eine Krankheit; Schädigung oder Tod von Neuronen innerhalb des verletzten Rückenmarks; und Quetschung von Axonen mit verletztem Rückenmark.

6. Verwendung gemäß Anspruch 5, wobei die Krankheit Polio, Spina bifida oder Friedreichs Ataxie ist.

7. Verwendung gemäß Anspruch 5, wobei die Neuronen motorische Neuronen sind.

8. Verwendung gemäß Anspruch 1, wobei die Menge an GASP für das Medikament 0,5 - 15 g pro kg Körpergewicht pro Tag beträgt.

9. Verwendung gemäß irgendeinem der vorangehenden Ansprüche zum Verbessern des Überlebens von Neuronen nach einer Rückenmarksverletzung.

10. Verwendung gemäß Anspruch 9, wobei das Neuron ein motorisches Neuron im verletzten Rückenmark ist.

11. Verwendung gemäß irgendeinem der vorangehenden Ansprüche zum Fördern der Axonregenerierung bei einer Rückenmarksverletzung.

12. Verwendung gemäß irgendeinem der vorangehenden Ansprüche, wobei die Behandlung eines Säugers mit Rückenmarksverletzung zum Fördern einer Proliferation und/oder einer Differenzierung neuronaler Stammzellen in einem Säuger ist.

13. Verwendung gemäß Anspruch 12, wobei die neuronalen Stammzellen zu irgendeiner der folgenden Zellarten differenziert werden:
Neuronen, Astrocyten; und Oligodendrocyten.

## Revendications

1. Utilisation de spores de Ganoderma (GASP) à germination activée dont le sporoderme est rompu, pour la préparation d'un médicament destiné au traitement d'un mammifère souffrant d'une lésion de la moelle épinière.

2. Utilisation selon la revendication 1, dans laquelle lesdits GASP sont destinés à être administrés par voie orale audit mammifère.

3. Utilisation selon la revendication 1, dans laquelle lesdits GASP sont destinés à être administrés audit mammifère dans le jour qui suit ladite lésion de la moelle épinière.

4. Utilisation selon la revendication 1, dans laquelle ledit mammifère est humain.

5. Utilisation selon la revendication 1, dans laquelle ladite lésion de la moelle épinière est due à l'une quelconque des conditions suivantes :
une compression ou une rupture de la moelle épinière ; un traumatisme ; une rupture d'une racine antérieure de la moelle épinière ; une rupture ou un écrasement du nerf sciatique ; une maladie ; un endommagement ou la mort de neurones à l'intérieur de ladite moelle épinière lésée ; et un écrasement d'axones à l'intérieur de ladite moelle épinière lésée.

6. Utilisation selon la revendication 5, dans laquelle ladite maladie est la poliomyélite, le spina-bifida, ou l'ataxie de Friedreich.

7. Utilisation selon la revendication 5, dans laquelle lesdits neurones sont des neurones moteurs.

8. Utilisation selon la revendication 1, dans laquelle la quantité desdits GASP pour ledit médicament est de 0,5 à 15 g par kg de poids corporel par jour.

9. Utilisation selon l'une quelconque des revendications précédentes, pour améliorer la survie de neurones après une lésion de la moelle épinière.

10. Utilisation selon la revendication 9, dans laquelle lesdits neurones sont des neurones moteurs dans ladite moelle épinière lésée.

11. Utilisation selon l'une quelconque des revendications précédentes, pour promouvoir la régénération d'axones lors d'une lésion de la moelle épinière.

12. Utilisation selon l'une quelconque des revendications précédentes, dans laquelle le traitement d'un mammifère souffrant d'une lésion de la moelle épinière a pour but de favoriser la prolifération et/ou la différenciation de cellules souches neurales chez un mammifère.

13. Utilisation selon la revendication 12, dans laquelle lesdites cellules souches neurales sont différenciées en l'un quelconque des types cellulaires suivants : neurones ; astrocytes ; et oligodendrocytes.
